# EUROPEAN PATENT SPECIFICATION

(11) **EP 3 043 180 B1**
(45) Date of publication and mention of the grant of the patent: **21.08.2019**
(21) Application number: 14842185.2
(22) Date of filing: 10.07.2014
(51) Int. Cl.: G01N 33/543, B01L 3/00

(54) **BIOCHIP**
BIOCHIP
BIOPUCE

(30) Priority: 06.09.2013 JP 2013185020
(43) Date of publication of application: 13.07.2016
(60) Divisional application: 19175763.2
(73) Proprietor: Nippon Light Metal Company, Ltd., Tokyo 140-8628 (JP)
(72) Inventor: MORISHITA, Ryo, Shizuoka-shi Shizuoka 421-3291 (JP); TAKEBAYASHI, Yasushi, Shizuoka-shi Shizuoka 421-3291 (JP); YAMAGUCHI, Kei, Shizuoka-shi Shizuoka 421-3291 (JP)
(74) Representative: Kador & Partner PartG mbB
(86) International application number: PCT/JP2014/068444
(87) International publication number: WO 2015/033668

(56) References cited:
- EP-A1- 1 457 553
- EP-A1- 1 486 786
- EP-A1- 2 306 197
- EP-A2- 1 284 495
- JP-A- 2004 097 200
- JP-A- 2010 008 378
- JP-A- 2011 101 623

## Description

### TECHNICAL FIELD

The present invention relates to a substrate for biochips, to which substrate biologically relevant substances such as proteins, nucleic acids, peptide derivatives, saccharides and derivatives thereof, natural products and small molecule compounds are immobilized as probes; and to a biochip comprising the same.

### BACKGROUND ART

Biochips such as protein chips, peptide chips and DNA chips are widely used for diagnosis and research of various diseases. The biochips, which have been widely used, are usually those obtained by immobilizing biologically relevant substances such as proteins, peptides and DNAs on a glass substrate such as a slide glass (for example, Patent Document 1, Patent Document 2 and the like).

In recent years, higher densification of biochips is demanded. In cases where biochips are highly densified, each reaction spot becomes small, and therefore the amount of the biologically relevant substances (which are expensive in many cases) immobilized on each spot can be reduced (see Patent Document 3). Further, if comprehensive analysis of genes or proteins can be conducted on a sheet of biochip, the procedures are simple and costs can be reduced, which is preferred.

### PRIOR ART DOCUMENTS

### Patent Document

Patent Document 1: JP 2006-329686 A
Patent Document 2: JP 2010-008378 A & EP 2 306 197
Patent Document 3: EP 1 457 553
Patent Document 4: EP 1 284 495

### SUMMARY OF THE INVENTION

### PROBLEMS TO BE SOLVED BY THE INVENTION

Although the present inventors made an effort to prepare biochips with high density, it was proved that in cases where the density of reaction spots is increased, various solutions of different biologically relevant substances used in forming each reaction spot are mixed with each other, and a biologically relevant substance(s) immobilized to each reaction spot is/are then likely to be contaminated (contamination).

An object of the present invention is to provide a substrate for biochips, in which contamination of a biologically relevant substance(s) to be immobilized does not occur even when reaction spots of a substrate for biochips are highly densified; and a biochip comprising the same.

### MEANS FOR SOLVING THE PROBLEMS

A possible way to avoid contamination of a biologically relevant substance(s) is providing a plurality of grooves, which surround each of reaction spots individually and independently, on the substrate. Such approach has been applied for the production of sample plates for mass spectrometry (see Patent Document 4).

Based on such approach, the present invention provides a biochip in which a biologically relevant substance(s) is/are immobilized on each of the reaction spots of the substrate for biochips, according to the appended claims.

### EFFECT OF THE INVENTION

The present invention provides biochips, in which reaction spots are highly densified but contamination of a biologically relevant substance(s) to be immobilized does.not occur; and therefore the accurate analysis is possible>. As a result, the size of each spot can be reduced and the amount of a biologically relevant substance(s), which is/are expensive, to be immobilized on each spot can be reduced.

### BRIEF DESCRIPTION OF THE DRAWINGS

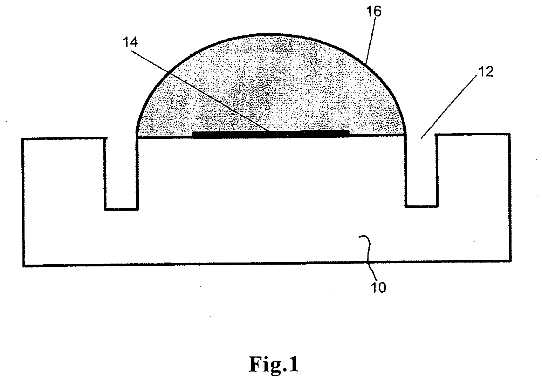

Fig. 1 is a schematic cross-sectional view showing a reaction spot, which is a specific example of a substrate for biochips of the present invention.

### MODE FOR CARRYING OUT THE INVENTION

A preferable specific example of the present invention will be explained based on Fig. 1, which is a schematic cross-sectional view of a reaction spot. In Fig. 1, 10 is a substrate, 12 is a groove, 14 is a reaction spot, and 16 is a droplet covering the reaction spot. These elements will now be described below.

Materials constituting the substrate 10 may be the same as those constituting known substrates for biochips, and examples of the materials include glass such as slide glass; plastics such as acrylic resin, polystyrene and polyethylene terephthalate; metals such as aluminum and stainless steel; carbon such as amorphous carbon and diamond-like carbon. Among these, carbon has excellent properties that autofluorescence is not induced, a biologically relevant substance(s) can be immobilized easily, processing of the substrate is easy, and high flatness and surface precision can be attained. Therefore, the biochip of the present invention comprises a substrate whose surface at least is composed of carbon is preferred. In order to promote the accuracy of measurement when used as a biochip, the surface of the substrate is preferably as flat as possible and may be polished as required. The surface roughness Ra is 2 nm or less, and preferably about 1 nm. Even though the substrate body is made of glass, metal, plastics or the like, the above-described excellent effects can be attained as long as the surface of the substrate is composed of carbon, and the substrate has flat surface.

For accurate analysis, it is preferable to bind a biologically relevant substance(s) covalently to the substrate more strongly to attain immobilization. For this reason, the surface of the reaction spot 14 preferably has a functional group(s) through a covalent bond, to covalently bind to a biologically relevant substance(s). Preferable examples of the functional group(s) to covalently bind to a biologically relevant substance(s) include amino groups and carboxyl groups, but the functional group(s) is/are not restricted thereto. Methods for binding amino groups or carboxyl groups covalently to the surface of the substrate composed of carbon are known (the above-described Patent Document I and Patent Document 2), and the amino groups and the carboxyl groups can be bound covalently to the surface of the substrate by these known methods. Preferably, an amino group-containing polymer or a carboxyl group-containing polymer is bound covalently to the surface of the substrate (Patent Document 2).

A plurality of reaction spots are present on the substrate, and the number of the reaction spots is usually not less than 300, preferably not less than 1000, more preferably about 10000 to 40000 per substrate having a size of of 25 mm × 75 mm slide glass.

On the substrate for biochips of the present invention, the groove 12, which surrounds each of the reaction spots individually and independently, is provided. The groove 12 surrounds each of the reaction spots individually. Also, each groove 12 surrounds each of the reaction spot 14 independently, that is, the grooves each surrounding a different reaction spot do not intersect or come into contact with one another. The width of the groove is 10 µm to 100 µm, preferably about 20 µm to 50 µm; and the depth of the groove is 0.5 µm to 50 µm, preferably about 1 µm to 20 µm. The planar shape of the groove 12 (the two-dimensional shape when the substrate is viewed from the top) is not restricted, but it may be a circle, square, rectangle, polygon, or the like. Preferably, the groove 12 may be formed easily by direct writing with a laserbeam. The size of the groove may be any size as long as the groove surrounds each reaction spot.

A biologically relevant substance(s) is/are immobilized to the reaction spots. The biologically relevant substance(s) may be any substance which is used as a probe in biochips, and examples thereof include any optional polypeptide (including natural or synthetic protein and oligopeptide), nucleic acid (including DNA and RNA, and artificial nucleic acid), saccharide, lipid, complex thereof (glycoprotein and the like), and derivative thereof (modified protein, nucleic acid and the like). In cases where a functional group(s) is/are added to the surface of the reaction spots, these biologically relevant substances are bound covalently to the functional groups by well-known methods.

When each of the biologically relevant substances is immobilized to each reaction spot of the substrate for biochips of the present invention, a solution containing a biologically relevant substance(s) (usually a solution containing a biologically relevant substance(s), surfactant, reagents and the like in aqueous buffer solution) is spotted (dropped). Fig. 1 shows the state schematically in which a solution is spotted. The spotted solution is spread on the whole surface of the inner side of the groove, and covers the reaction spot 14 completely to form a thickly-raised droplet 16. After maintaining this state, the biologically relevant substance(s) in the droplet is/are immobilized to the reaction spot. In this case, although a solution containing the biologically relevant substance(s) and surfactant has a smaller surface tension than that of pure water, the peripheral portion of the droplet 16 is caught at the upper edge of the inner side of the groove 12, and does not to enter the interior of the groove 12 as shown in Fig. 1, thereby keeping the raised shape of the droplet 16 as shown in Fig. Owing to this, each droplet of adjacent reaction spots is not mixed each other, and therefore, contamination of the biologically relevant substance(s) to be immobilized does not occur. The droplet 16 does not evaporate easily because it is maintained in a raised state as shown in Fig., and the volume is large, which can maintain the immobilized biologically relevant substance(s) in wet state for a long time. Although a biologically relevant substance such as protein is denatured and inactivated due to drying in most cases, the biochips of the present invention can maintain a biologically relevant substance(s) placed on each spot in wet state for a long time, which is advantageous.

The thus obtained biochip may be used in exactly the same manner as in the conventional biochips.

The present invention will now be described more concretely by way of Examples. However, the present invention is not restricted to the Examples below.

### EXAMPLE

### 1. Production of Substrate for Biochips

Using, as a substrate material, an amorphous carbon plate (25.0 × 75.0 mm, tolerance ±0.1 mm, plate thickness 1.000 mm, tolerance ±0.025 mm) which was polished such that the surface roughness Ra was 1 nm, a 15-minute ultraviolet irradiation (18.5 mW/cm², 254 nm) was performed by an ultraviolet irradiation apparatus (SEN LIGHTS Co., Ltd., Photo Surface Processor PL16-110).

The reaction spots had a diameter of 1.5 mm, and 1536 reaction spots were formed. Around each reaction spot, grooves each surrounding the reaction spot were formed respectively by direct writing with a laserbeam (Apparatus: MDV9600A, produced by KEYENCE CORPORATION, Output 200 kW). Each groove had a diameter of about 2 mm, a depth of about 10 µm and a width of about 40 µm.

Amino groups were bound covalently on the surface of the reaction spots as follows:
1. The substrate was subjected to scrub washing and drying with a spin dryer.
2. The substrate was irradiated with UV having a wavelength of 185 nm/235 nm in the air for 5 minutes.
3. The substrate was coated with a solution of polyallylamine (PAA) in ethanol with a Baker applicator
4. The coated substrate was incubated at high humidity of 95% Rh for 15 minutes.
5. A 15-minute vacuum drying was performed under reduced pressure of not more than 0.095 MPa.
6. A 3-minute UV irradiation was performed under reduced pressure of not more than 0.095 MPa to immobilize PAA.
7. The substrate was washed by performing a 5-minute immersion in pure water twice, and dried with a spin dryer for 40 seconds.

### 2. Production of Biochip

Next, mKate protein was immobilized to each reaction spot by a covalent bond. This procedure was carried out concretely in the following way. The substrate, which was grooved and modified with amino groups, was immersed in 0.2 mM Sulfo-SMPB (Thermo scientific) solution which was a cross-linker. After adding maleimide groups to each reaction spot, the substrate was immersed in 50 mM solution of GSH having a thiol group to bind GSH to the maleimide group. The mKate protein was synthesized in the form having FLAG and GST tags added in the N-terminal side, by using an extract of wheat germ. A solution of FLAG-GST-mKate protein was then spotted to the spot, to which GSH was bound, using automatic pipettor, thereby allowing GSH and GST to react to bind each other. When the mKate protein solution was spotted to each reaction spot, as schematically shown in Fig. 1, the droplet 16 was spread on the inner side of the groove, and the peripheral portion was caught at the upper edge of the groove 12 not to enter the internal of the groove 12. Therefore, the droplet did not run off the edge, and the raised shape of the droplet 16 was kept as shown in Fig.

### 3. Evaluation

The above-described binding of the mKate protein was confirmed by reacting Anti-FLAG-HyLight-647 and measuring fluorescence intensity. As a result, independent fluorescences of each spot could be detected due to the effect of the groove, and the binding of the mKate protein on the whole surface in a reaction spot could be confirmed.

### DESCRIPTION OF SYMBOLS

- 10: Substrate
- 12: Groove
- 14: Reaction spot
- 16: Droplet

## Claims

1. A biochip in which a biologically relevant substance(s) selected from the group consisting of polypeptides, nucleic acids, saccharides, lipids, a complex thereof and a derivative thereof which biologically relevant substance(s) is/are immobilized as probes on each of a plurality of reaction spots (14) disposed on a substrate for biochips,
wherein said substrate comprises a substrate (10) and a plurality of reaction spots (14) disposed on the substrate, wherein a plurality of grooves (12) which surround each of said reaction spots (14) individually and independently are provided on the substrate (10), and the grooves (12) each surrounding a different reaction spot (14) do not intersect or come into contact with one another, said said grooves (12) having a width of 10 µm to 100 µm and a depth of 0.5 µm to 50 µm,
wherein said biologically relevant substance(s) is/are provided in a droplet (16), and a peripheral portion of the droplet (16) is caught at the upper edge of the inner side of the groove (12) and does not enter the interior of the groove (12),
the surface of the substrate has a surface roughness Ra of 2 nm or less, and
at least the surface of said substrate (10) is composed of carbon.

2. The biochip according to claim 1, wherein a functional group(s) is/are immobilized to each of the reaction spots (14) by a covalent bond.

3. The biochip according to claim 2, wherein said functional group(s) is/are an amino group and/or a carboxyl group.

4. Use of a biochip according to any one of claims 1 to 3, wherein said biologically relevant substance(s) is/are used as probe(s).

## Patentansprüche

1. Biochip, in dem eine biologisch relevante Substanz(en), ausgewählt aus der Gruppe bestehend aus Polypeptiden, Nukleinsäuren, Sacchariden, Lipiden, einem Komplex davon und einem Derivat davon, wobei die biologisch relevante(n) Substanz(en) als Sonden auf jedem von einer Vielzahl von Reaktionsstellen (14) immobilisiert ist/sind, die auf einem Substrat für Biochips angeordnet sind,
wobei das Substrat ein Substrat (10) und eine Vielzahl von Reaktionsstellen (14) umfasst, die auf dem Substrat angeordnet sind, wobei eine Vielzahl von Vertiefungen (12), die jede der Reaktionsstellen (14) einzeln und unabhängig umgeben, auf dem Substrat (10) bereitgestellt sind, und die Vertiefungen (12), die jeweils eine andere Reaktionsstelle (14) umgeben, sich nicht durchschneiden oder nicht miteinander in Kontakt kommen, wobei die Vertiefungen (12) eine Breite von 10 µm bis 100 µm und eine Tiefe von 0,5 µm bis 50 µm aufweisen,
wobei die biologisch relevante(n) Substanz(en) in einem Tröpfchen (16) bereitgestellt ist/sind, und ein peripherer Teil des Tröpfchens (16) an der Oberkante der Innenseite der Vertiefung (12) gefangen ist und nicht in das Innere der Vertiefung (12) eindringt,
wobei die Oberfläche des Substrats eine Oberflächenrauheit Ra von 2 nm oder weniger aufweist und
wobei mindestens die Oberfläche des Substrats (10) aus Kohlenstoff besteht.

2. Biochip nach Anspruch 1, wobei eine funktionelle Gruppe(n) durch eine kovalente Bindung an jeder der Reaktionsstellen (14) immobilisiert ist/sind.

3. Biochip nach Anspruch 2, wobei die funktionelle(n) Gruppe(n) eine Aminogruppe und/oder eine Carboxylgruppe ist/sind.

4. Verwendung eines Biochips nach einem der Ansprüche 1 bis 3, wobei die biologisch relevante(n) Substanz(en) als Sonde(n) verwendet wird/werden.

## Revendications

1. Biopuce contenant une/des substance(s) biologiquement pertinente(s) sélectionnée(s) dans le groupe consistant en des polypeptides, des acides nucléiques, des saccharides, des lipides, un complexe de ceux-ci et un dérivé de ceux-ci, laquelle/lesquelles substance(s) biologiquement pertinente(s) est/sont immobilisée(s) en tant que sondes sur chacun d'une pluralité de spots de réaction (14) disposés sur un substrat pour biopuces,
dans laquelle ledit substrat comprend un substrat (10) et une pluralité de spots de réaction (14) disposés sur le substrat, où une pluralité de sillons (12) qui entourent chacun desdits spots de réaction (14) sont individuellement et indépendamment mis à disposition sur le substrat (10), et les sillons (12) entourant chacun un spot de réaction différent (14) ne se croisent pas ou ne sont pas en contact les uns avec les autres, lesdits sillons (12) possédant une largeur de 10 µm à 100 µm et une profondeur de 0,5 µm à 50 µm,
dans laquelle ladite/lesdites substance(s) biologiquement pertinente(s) est/sont mise(s) à disposition dans une gouttelette (16), et une partie périphérique de la gouttelette (16) est retenue au niveau du bord supérieur de la face interne du sillon (12) et n'entre pas à l'intérieur du sillon (12),
la surface du substrat possède une rugosité de surface Ra de 2 nm ou moins, et
au moins la surface dudit substrat (10) est composée de carbone.

2. Biopuce selon la revendication 1, dans laquelle un/des groupe(s) fonctionnel(s) est/sont immobilisé(s) à chacun des spots de réaction (14) par une liaison covalente.

3. Biopuce selon la revendication 2, dans laquelle ledit/lesdits groupe(s) fonctionnel(s) est/sont un groupe amino et/ou un groupe carboxyle.

4. Utilisation d'une biopuce selon l'une quelconque des revendications 1 à 3, dans laquelle ladite/lesdites substance(s) biologiquement pertinente(s) est/sont utilisée(s) en tant que sonde(s).
